# EUROPEAN PATENT APPLICATION

(11) **EP 3 708 140 A1**
(43) Date of publication of application: **16.09.2020**
(21) Application number: 19161952.7
(22) Date of filing: 11.03.2019
(51) Int. Cl.: A61K 6/083, A61K 6/06, A61K 6/02, A61K 6/00

(54) **POLYMERIZABLE BIOACTIVE COMPOSITIONS**

(71) Applicant: S & C Polymer Silicon- und Composite-Spezialitäten GmbH, 25335 Elmshorn (DE)
(72) Inventor: Engelbrecht, Juergen, 25335 Elmshorn (DE)
(74) Representative: Forstmeyer, Dietmar

(57) **Abstract**

The present invention refers to dental bioactive compositions. Such compositions can contain a resin portion having at least one polymerizable unsaturated monomer or oligomer and a carboxylate of a multivalent cation, and another portion containing at least one hydraulic filler. The compositions can be used as dental pulp lining or capping material.

## Description

The present invention refers to dental bioactive compositions. Such compositions can contain a resin portion having at least one polymerizable unsaturated monomer or oligomer and a carboxylate of a multivalent cation, and another portion containing at least one hydraulic filler. The compositions can be used as dental pulp lining or capping material.

### Background Art

Among the three major structures of the tooth, enamel, dentin and the soft tissue pulp, both dentin and pulp are considered vital and sensitive. Although enervation and blood supply are encountered only in the pulp, dentin is physiologically linked to the pulp by the dentinal fluid that permeates the entire structure of dentin within the dentinal tubules. Because of the hydraulic connection any injury affecting the external portion of the tooth will immediately be sensed by the pulp, which will respond in a defensive manner against the attack. The more the injury (trauma, caries) approaches the confined chamber of the pulp, the more the pulp requires professional intervention to aid its intrinsic ability to heal. Professional intervention should be directed to not only remove the aggressive element, but also to help pulp healing and protect the dentin pulp complex against further injury.

Bioactive dental materials refer to materials that have a biological effect on surrounding tissues. For example, a bioactive liner may cause the release of growth factors from the dentinal collagen to induce the formation of reparative dentin in the pulp chamber. A desirable bioactive restorative material creates a bond with surrounding tooth structure, and releases ions to allow remineralization at tooth margins following an acid challenge. A bioactive cement may have the additional benefit of attracting calcium phosphate precipitates to its surface to occlude an existing cement gap.

Bioactive materials utilized in dentistry fall primarily in two compositional classes: calcium silicates and calcium aluminates. Both materials, when their powder component is mixed with water, set with an acid-base chemical reaction and produce an alkaline pH after setting. The most active of the bioactive materials demonstrate a high pH level in storage solutions. High pH levels (7.5 or higher) appear to stimulate more active and complete bioactivity. They can form a layer of calcium phosphate apatite and calcium phosphate precipitates at the interface between the bioactive restorative material and dentin and release calcium, phosphate and fluoride ions.

A first bioactive material Calcium Hydroxide [Ca(OH)₂] was reported early on as a composition useful in vital pulp therapy and as a protective agent for the so-called dentin/pulp complex. Its ability to trigger regeneration of pulp tissue was first described in 1930 [Hermann B W, Dentinobliteration der Wurzelkanäle nach Behandlung mit Calcium, Zahn Rundschau 39, 888-899, 1930]. When applied directly over the exposed pulp tissue in a pure powder or aqueous solution, Ca(OH)₂ was reported to cause an immediate surface, chemical cauterization that destroys part of the tissue. This feature led researchers to seek formulations that could induce healing without sacrificing the remaining tissue, starting with powdered formulations of Ca(OH)₂ that were admixed with water prior to application to the pulp or dentin. Although the extract mechanism by wich Ca(OH)₂ generates pulp healing by the induction of dentin bridge formation (formation of a new, reparative dentin that occludes the pulp exposure site and separates it from the external environment, thus enclosing the exposed pulp again in the chamber) is not fully understood, it is well accepted that the high pH of its formulations (ca. 11 - 13) has been regarded as one, if not the major factor. Stanley, Criteria for standardizing and increasing credibility of direct pulp capping studies [Am J Dent 11 Sp. Iss (1998).

A commercial two component paste/paste Ca(OH)₂ based pulp liner formulation is named Dycal currently marketed by Dentsply. A single component/visible light cure formulation also employing Ca(OH)₂ is commercially marketed by Dentsply under the name PRISMA® VLC™ Dycal®.

However, studies suggest that a hermetic seal of the exposed or nearly exposed pulp chamber is more important to long term pulp survival than the use of calcium hydroxide when the pulp cavity has been exposed, or when the dentin layer covering the pulp cavity is nearly breached. See Kopel H.; J. of Dent. Child; September-October 7997; The Pulp Capping Procedure in Primary Teeth "Revisited." Further, calcium hydroxide is a highly soluble form of calcium that readily washes out with pulpal fluid allowing its bacteriostatic properties to become nonexistent before the dental pulp is allowed to regenerate dentin and dentin bridges to produce a new pulp cavity. Finally, calcium hydroxide interferes with bonding of resin-based dental restorations to the underlying dentin, and the high pH of calcium hydroxide can further neutralize the acid etching process used to help ensure a strong bond of the restoration to the underlying tissue, especially since calcium hydroxide compositions are typically added over the pulp cap prior to using a final restorative material such as a resin composites, glass ionomers, or amalgam. Due to these problems, restorations produced by using a calcium hydroxide or calcium hydroxide-based formulation liner often fail or must be recreated due to poor bonding or erosion of the liner, allowing bacteria to invade the pulp cavity, requiring the patient to return to the clinician to have the tooth removed or the restoration replaced.

In response to these shortcomings, methods of using restoration materials consisting of Portland cements have been proposed. The relative advantages of such cement formulations are that its diffusion to the soft, exposed pulp is limited and the chemical destruction of the sound tissue is minor. Additionally, the cement formulation is significantly less soluble and is expected to last longer underneath the restorations as well as to provide higher mechanical strength and to allow the material to be placed as a liner underneath restorations and support the load during application (in case of amalgams that are condensed) and subsequent mastication. For example, U.S. Pat. Nos. 5,415,547 and 5,769,638 to Torabinejad et al. suggest the use of a traditional or fast-set Portland cement having a calcium component of about 50-70% and a silicon dioxide component of about 21% as a filling material, with said material setting when hydrated. However, as noted in those references, the Portland cement filling compositions require traditional filling materials to be used as the final permanent restorative as Portland cement does not have sufficient wear properties. Further, a temporary filling must be used for the first 24 hours after the cement filling or pulp cap has been applied requiring a patient to return to the dentist the next day to have the temporary filling removed and permanent filling inserted as the wear surface once the cement has hardened. These drawbacks, coupled with the difficulty to predict the working time of the filling material, make these compositions and methods logistically less desirable than traditional methods.

Currently available calcium-containing lining and capping materials are designed to be applied as both indirect and direct pulp capping agent. Direct pulp capping indicates application direct on the exposed pulp surface. In this case, the alkalinity of the material acts immediately on the tissue and triggers the pulp cells towards formation of new calcified tissue to close the exposure. Indirect pulp capping indicates application of the material on dentin surface, not in direct contact with the pulp. In this case, professional intervention occurred before the pulp became exposed and the calcium-containing material is mostly used for its protective effect against further injury that may migrate to the pulp through the dentinal tubules. A calcium-containing layer applied as a liner on the deepest part of the cavity will form a physical barrier against the intrusion of potentially aggressive agents into dentinal tubules. Additionally, it is expected that such calcium-containing materials will promote a distant healing effect on the pulp, even though not in direct contact with it. This is reportedly because the calcium content of the materials is released and that alkaline ions can travel across the remaining dentin thickness and reach the pulp to deliver its benefits. It is well understood however, that release of calcium occurs at the expense of the solubilization of the material. Ca(OH)₂-based cements are set hard through a simple acid-base reaction and this ionically-hardened material is indeed very soluble. However, a recent clinical study reports that the aforementioned Dycal® material comprising Ca(OH)₂, seemed to disappear under amalgam restorations over the years due to solubilization of the material induced by oral fluids migrating from marginal leakage. Pereira et al., Clinical evaluation of Dycal under amalgam restorations, Am l.Dent 3:67 -70. Some believe that this solubility is necessary for prolonged activity of the alkaline effect. One benefit of Ca(OH)₂, that is considered additional to its ability to protect and heal dentin/pulp complex is its antibacterial property. Bacteria can not survive at such a high pH resulting from localized release of OH ions.

Other reported major shortcomings of Ca(OH)₂, as a direct or indirect pulp capping material alone or in cement formulations are that the strength of the material is low and its solubility over time leaves a gap underneath the restorations leading to sensitivity and potential microbial growth as the alkaline benefits are no longer there once the material is dissolved. Another major drawback is that such cements are not adhesive and do not improve sealing of dentin. Depending on the amount applied to cover dentin underneath an adhesive restoration, all the covered area will no longer be available for the bonding procedure and the overall retention and sealing may be compromised. Investigations into alternatives to traditional Ca(OH)₂ cements have lead to attempts to produce resin-based cements or lining or capping systems wherein one or more ethylenically unsaturated monomers are employed with Ca(OH)₂, or Portland cements or other flller compounds to create two component systems or one component systems capable of light curing. See, e.g. Lopez et al. US Published Application No. 20020045678: Yudha et al. U.S. Pat. No. 6,032,832; and PCT Published Appiication WO 01/12129 A1. The cement systems disclosed in Yudha et al. includes polymerizable unsaturated dicarboxylic acid compounds, (methyl) methacrylates copolymers such as Bis-GMA, and a metal chelate-forming inorganic powder. Yudha et al. characterize their systems as practically non-aqueous cement in which the resin component comprises a polymerizable monomer such as 2-hydroxyethyl methacrylate (2-HEMA) which is used as a reactive solvent to dissolve a carboxyl group-containing polymer along with an inorganic powder that forms a metal chelate in the presence of water. The root canal sealants and fillers and pulp capping material disclosed in WO 01/012129 A1 include biodegradable polymers, copolymers comprising acrylates and methacrylates such as 2-HEMA and triethylene glycol trimethacryiates.
Lopez et al. disclose dental restorative compositions comprising an unsaturated resin portion that can comprise the aforementioned WO 01/0129129 A1 biodegradable resins as well as other resin compounds such as traditional BisGMA compounds and a variety of acidic monomers such as DSDM, BPDM, a calcium silicate cement component and a non-water curing component (see Lopez et al.., paragraphs 009-014), together with fillers and other components such as radiopaque materials such as barium sulphate and bismuth oxide.

However, attempts to follow the suggestions in the art have revealed that certain problems may exist. For example, use of BisGMA with acidic monomers such as DSDM as suggested in Lopez et al. but in the presences of relatively higher concentrations (above about 40 wt. percent) of Portland Cement III reveals that the one component systems gels and is not shelf-stable for long periods of time. Additionally, efforts to mix monomers such as BisGMA, triethylene glycol dimethacrylate (TriEDMA) with barium sulfate powders and Portland cements resulted in separation of the composition into different components, which was
confirmed by microscopic examination of the miscibility of the composition in water. Removal of BisGMA and other conventional polymerizable monomers such as UDMA from the system, and attempts to cure polymerizable monomers such as the polyethylene glycol dimethacrylate (PEGDMA) taught by Lopez et al. showed very low bond strengths after curing.

Suh et al. described in US 2008/0318190 the balance of hydrophobic and hydrophilic portions to overcome problems of initially hydrophilic characteristics for faster interaction with the tooth and subsequent hydrophobic characteristics to maintain strength while allowing prolonged Ca²⁺- and OH⁻ -Ion release to promote pulp and dentin vitality and healing. Nevertheless, the most effective pulp capping and dentin protecting materials are the hydraulic cements like MTA/Portland cement itself. They show more and faster calcium release and faster tertiary dentin building (healing).

It is the object of the present invention to overcome the above-mentioned drawbacks of the prior art materials.

Acording to the present invention, there is provided a polymerizable mixture comprising
a.) a compound of formula (I)

   (RCOO⁻)ₓM^{x+} (I)

   wherein each R may be the same or different and is a polymerizable, optionally substituted hydrocarbon group, and each M may be the same or different and is a multivalent metal, and
b.) at least one hydraulic filler.

Surprisingly, it was found that the incorporation of metal salt monomers or oligomers into e.g. pulp capping and dentin lining mixtures containing hydraulic cements are able to accelerate and increase the initial and subsequent release of Ca²⁺ - and OH⁻ -ions thereby promoting pulp and dentin vitality and healing, while enhancing the strength of the polymerized mixtures. Moreover, curing of the products can be accelerated.

The products obtained according to the present invention moreover have an extended shelf life and show basically no separation into different components.

In the above formula (I), at least one R can contain two, three or more polymerizable groups.

Preferably all Rs can contain two, three or more polymerizable groups.

In a preferred embodiment, the polymerizable groups are unsaturated hydrocarbon groups which can be optionally substituted.

In a preferred embodiment the hydrocarbon group is an optionally substituted C₂-C₁₀ straight or branched chain alkyl group comprising at least one, preferably 2, 3 or 4 CC double bonds.

The hydrocarbon group can be unsubstituted or can have 1, 2, 3, 4, 5 or more substituents.

The substituents can be selected from C₁-C₃ alkyl or C₂-C₄ alkenyl groups, phenyl or benzyl groups, -OH, -NH₂, -NO₂ or -SH groups, whereof C₁-C₃ alkyl groups, especially methyl groups, and unsubstituted benzyl groups are preferred.

These polymerizable groups can, for example, be selected from one or more groups of the formulae

CH₂=CR'-[CH=CR'-]ₙ-,

CH₂=CR'-[CH₂-CHR'-]ₙ-,

CHR"=CH- [CR"=CH-]ₙ-,

CHR"=CH- [CHR"-CH₂-]ₙ-,

*HOOC-[CH2]ₙ*-*CH*=*CH-,*

*CH2=CH-C6H4-,*

wherein R' is a hydrogen atom or a methyl group,
R" is a benzyl group, and
n is 0, 1, 2, 3 or 4,
or oligomers thereof.

Especially preferred n is = or 1.

According to a preferred embodiment, R is the residue of vinyl benzoic acid, or of reaction products of maleic, fumaric or succinic acid anhydride or aromatic 4-MET anhydrides (e. g. trimellitic acid) with hydroxyethyl methacrylates.

In the present invention, M can for example be a divalent metal, preferably Mg²⁺, Ca²⁺, Sr²⁺ and / or Zn²⁺, or a trivalent metal, preferably Al³⁺ and / or Fe³⁺.

According to an especially preferred embodiment, M is Ca²⁺ and R is a monomethacryloyl oxyethyl maleic acid residue.

The mixtures according to the present invention can additionally contain another polymerizable unsaturated compound, preferably methacrylate monomers or oligomers or acrylate monomers or oligomers and/or mixtures thereof.

In the mixtures according to the present invention the compound of formula (I) is for example present in an amount of about 1 - 99 wt.-%, preferably of about 1 - 40 wt.-%, especially preferred in an amount of about 2 - 10 wt.-%, based on the total weight of the polymerizable mixture.

The hydraulic filler b.) can be a metal silicate, metal aluminate, metal oxide, metal hydroxide, bioglass, or bioceramic, like a calcium silicate, a calcium aluminate, a calcium oxide, calcium hydroxide, bioglass 45S5, a ceramic bioglass, or a mixture thereof.

It may be preferable in terms of flowability and reactivity that the average grain size (D50) of the hydraulic filler is not greater than 5 microns, especially preferred not greater than 3 microns.

The mixtures of the present invention can also contain a mixture of two, three or more hydraulic fillers.

The filler can be present in an amount of about 1 - 90 wt.-%, preferably of 30 - 85 wt.-%, based on the total mixture.

In a further preferred embodiment, the mixtures can also contain additional polymerizable unsaturated monomers or oligomers. These additional components can be present in an amount of 5 to 30 wt.-%, preferably 10 to 20 wt.-%, based on the total mixture.

In a further preferred embodiment, the mixtures can also contain one or more additional non-hydraulic inorganic or organic fillers or mixtures thereof. These additional fillers can be present in an amount of 5 to 30 wt.-%, preferably 10 to 20 wt.-%, based on the total mixture.

The mixtures according to the present invention can be used as a pulp capping material, a liner, a dental base material, a cavity lining, a dentin replacement material, a cement for crowns, a cement for bridges, a root canal filling material, an adhesive for brackets or a bone cement.

The mixtures according to the present invention are preferably configured to be pastes having a flowability that is appropriate for dental applications.

According to a preferred embodiment, the inventive mixtures are light curable; thereby they can be cured very fast and result in products with improved mechanical properties.

According to a preferred embodiment, the inventive mixtures can be dual curable mixtures.

They can also contain usual additives like radiopaquers (like bismuth oxide) etc.

The invention will now be described by the following examples, it is, however, not limited by the examples:

### Examples

All amounts indicated are wt.% unless indicated otherwise.

### Preparation of a Ca-salt of Monomethacryloyloxyethylmaleic acid (MAOEM)

20 g Ca(OH)₂ are reacted at room temperature with 125 g MAOEM in ethyl acetate while stirring (∼ 4h), until the Ca(OH)₂ has reacted completely. Then the solvent is removed under reduced pressure. A calcium salt is obtained with a highly viscous resin-like consistence. (furthermore called "Ca-MAOEM").

### Comparative Example

The following components are combined and mixed at room temperature until full homogeneity is achieved:

**"UB-0"**

| | |
|---|---|
| UDMA (Urethanedimethacrylate) | 20.0 |
| EBADMA (Ethoxylated Bisphenol A Dimethacrylate) | 20.0 |
| Ca-MAOEM | 0.0 |
| Bioglass 45S5 | 45.0 |
| Barium sulfate | 10.0 |
| Silicon dioxide | 4.5 |
| Camphorquinone | 0.2 |
| Aminobenzoic acid | 0.3 |

A light curable product is obtained.

### Example 1

The following components are combined and mixed at room temperature until full homogeneity is achieved:

**"UB-5"**

| | |
|---|---|
| UDMA | 17.5 |
| EBADMA | 17.5 |
| Ca-MAOEM | 5.0 |
| Bioglass 45S5 | 45.0 |
| Bariumsulfate | 10.0 |
| Silicon dioxide | 4.5 |
| Camphorquinone | 0.2 |
| Aminobenzoic acid | 0.3 |

A light curable product is obtained.

### Example 2

The following components are combined and mixed at room temperature until full homogeneity is achieved:

**"UB-10"**

| | |
|---|---|
| UDMA | 15.0 |
| EBADMA | 15.0 |
| Ca-MAOEM | 10.0 |
| Bioglass 45S5 | 45.0 |
| Bariumsulfate | 10.0 |
| Silicon dioxide | 4.5 |
| Camphorquinone | 0.2 |
| Aminobenzoic acid | 0.3 |

### Example 3

The following components are combined and mixed at room temperature until full homogeneity is achieved:

**"UB-20"**

| | |
|---|---|
| UDMA | 10.0 |
| EBADMA | 10.0 |
| Ca-MAOEM | 20.0 |
| Bioglass 45S5 | 45.0 |
| Bariumsulfate | 10.0 |
| Silicon dioxide | 4.5 |
| Camphorquinone | 0.2 |
| Aminobenzoic acid | 0.3 |

A light curable product is obtained.

The products obtained were cured with light for 20 s to obtain test specimens.

### Results:

**Release of Ca²⁺- Ions- (1 week) [ppm]**

| | |
|---|---|
| UB-0 | 0 |
| UB-5 | 3 |
| UB-10 | 21 |
| UB-20 | 225 |

The amount of Ca²⁺- Ions was determined in a manner known to a person skilled in the art by complexometric tiration using Titriplex (EDTA), Erichrome Black T and an ammonia buffer.

**Barcol hardness (24 h)**

| | |
|---|---|
| UB-0 | 56 |
| UB-5 | 68 |
| UB-10 | 66 |
| UB-20 | 68 |

The Barcol hardness was measured in a manner known to a person skilled in the art according to DIN EN 59 using a digital HPE II Barcol tester.

It can be taken from the examples that the higher the content of Ca-MAOEM in the mixtures according to the present invention, the better the release of Ca²⁺ ions of the test specimens. Moreover, also the hardness of the test specimens was improved.

In the following Examples and Comparative Example, Mixtures containing tricalcium silicate, dicalcium silicate, and tricalcium aluminum silicate in a weight ratio of 3:2:1 in a hydrophilic resin matrix were used (furthermore called "MTA")

### Comparative Example

The following components are combined and mixed at room temperature until full homogeneity is achieved:

**"MTA-0"**

| | |
|---|---|
| UDMA | 15.0 |
| HEMA (Hydroxylethylmethacrylate) | 5.0 |
| PEG-400-DMA (Polyethylenglycole Dimethacrylate) | 20.0 |
| Ca-MAOEM | 0.0 |
| MTA | 45.0 |
| Bariumsulfate | 10.0 |
| Silicon dioxide | 4.5 |
| Camphorquinone | 0.2 |
| Aminobenzoic acid | 0.3 |

### Example 4

The following components are combined and mixed at room temperature until full homogeneity is achieved:

**"MTA-5"**

| | |
|---|---|
| UDMA | 15.0 |
| HEMA | 5.0 |
| PEG-400-DMA | 15.0 |
| Ca-MAOEM | 5.0 |
| MTA | 45.0 |
| Bariumsulfate | 10.0 |
| Silicon dioxide | 4.5 |
| Camphorquinone | 0.2 |
| Aminobenzoic acid | 0.3 |

### Example 5

The following components are combined and mixed at room temperature until full homogeneity is achieved:

**"MTA-20"**

| | |
|---|---|
| UDMA | 15.0 |
| HEMA | 5.0 |
| PEG-400-DMA | 0.0 |
| Ca-MAOEM | 20.0 |
| MTA | 45.0 |
| Bariumsulfate | 10.0 |
| Silicon dioxide | 4.5 |
| Camphorquinone | 0.2 |
| Aminobenzoic acid | 0.3 |

A light curable product is obtained.

The products were cured with light for 20 s to obtain test specimens.

### Results:

**Release of Ca²⁺- Ions- (1 week) [ppm]**

| | |
|---|---|
| MTA-0 | 51 |
| MTA-5 | 85 |
| MTA-20 | 380 |

The amount of Ca²⁺- Ions was determined in a manner known to a person skilled in the art by complexometric tiration using Titriplex (EDTA), Erichrome Black T and an ammonia buffer.

**Barcol hardness (24 h)**

| | |
|---|---|
| MTA-0 | 51 |
| MTA-5 | 62 |
| MTA-20 | 73 |

The Barcol hardness was measured in a manner known to a person skilled in the art according to DIN EN 59 using a digital HPE II Barcol tester.

It can be taken from the examples that the higher the content of Ca-MAOEM in the mixtures according to the present invention, the better the release of Ca²⁺ ions of the test specimens. Moreover, also the hardness of the test specimens was improved.

To compare the properties of the products, obtained with mixtures according to the present invention, with prior art products, the following tests were carried out:
First, the release of Ca²⁺ ions of a commercially available resin-free MTA cement (Harvard MTA Universal, Harvard Dental International GmbH, Deutschland) and a commercially available resin-modified light-curable MTA cement (TheraCal LC, BISCO, USA) were measured.

Resin-free MTA cement was used since it is considered to have an optimal release of Ca²⁺ ions and therefore optimal remineralization properties.

Historically, for decades products like "Dycal" (Dentsply Caulk, USA), had been considered to be well-established hardening Calcium hydroxide liners creating tertiary dentine. Therefore, it was used as a further comparative product.

**Release of Ca²⁺- Ions- (1 week) [ppm]**

| | |
|---|---|
| Harvard MTA Universal | 375 |
| TheraCal LC | 105 |
| Dycal | 94 |

Resin-modified MTA formulations are acceptable but their potential for Ca²⁺- Ion release and thereby their potential for remineralization is markedly inferior to that of resin-free MTA-formulations.

The resin-modified mixtures of the present invention have a potential for Ca²⁺- Ion release comparable or more close to that of resin-free MTA cements and are significantly improved over the resin-modified MTA cements of the prior art.

Moreover, the mixtures according to the present invention have a very low solubility and can be used either as light curing or dual curing (two component) mixtures for:
Pulp capping material
Liner
Basis material / cavity lining
Dentin replacement
Cement for crowns
Cement for bridges
Root canal filling material
Adhesive for brackets
Bone cement

Note: Resin modified MTA-formulations have the advantageous property that they can be cured faster.

## Claims

1. A polymerizable mixture comprising
c.) a compound of formula (I)
(RCOO-)ₓM^{x+} (I)
wherein each R may be the same or different and is a polymerizable, optionally substituted hydrocarbon group, and
each M may be the same or different and is a multivalent metal, and
b.) at least one hydraulic filler.

2. A mixture according to claim 1, wherein at least one R, preferable all Rs contain two, three or more polymerizable, preferably unsaturated, groups.

3. A mixture according to claims 1 or 2, wherein R can be selected from one or more groups of the formulae
CH₂=CR'-[CH=CR'-]ₙ⁻,
CH₂=CR'-[CH₂-CHR'-]ₙ-,
CHR"=CH-[CR"=CH-]ₙ-,
CHR"=CH-[CHR"-CH₂-]ₙ-,
*HOOC-[CH₂]ₙ-CH*=*CH-,*
*CH₂*=*CH-C₆H₄*-,
wherein R' is a hydrogen atom or a methyl group,
R" is a benzyl group, and
n is 0, 1, 2, 3 or 4,
or oligomers thereof.

4. A mixture according to claim 1 wherein R is the residue of vinyl benzoic acid, of reaction products of maleic, fumaric or succinic acid anhydride or aromatic 4-MET anhydrides (e. g. trimellitic acid) with hydroxyethyl methacrylates.

5. A mixture according to any preceeding claim, wherein M is a divalent metal, preferably Mg²⁺, Ca²⁺, Sr²⁺ and / or Zn²⁺, or a trivalent metal, preferably Al³⁺ and / or Fe³⁺.

6. A mixture according to any preceeding claim, wherein M is Ca²⁺ and R is a monomethacryloyl oxyethyl maleic acid residue.

7. A mixture according to any preceeding claim, additionally containing another polymerizable unsaturated compound, preferably methacrylate monomers or oligomers or acrylate monomers or oligomers and/or mixtures thereof.

8. A mixture according to any preceeding claim, wherein the compound of formula (I) is present in an amount of about 1 - 99 wt.-%, preferably of about 1 - 40 wt.-%, especially preferred in an amount of about 2 - 10 wt.-%, based on the total weight of the polymerizable mixture.

9. A mixture according to any preceeding claim, wherein the hydraulic filler b.) is a metal silicate, metal aluminate, metal oxide, metal hydroxide, bioglass, or bioceramic, like a calcium silicate, a calcium aluminate, a calcium oxide, calcium hydroxide, bioglass 45S5, a ceramic bioglass, or a mixture thereof.

10. A mixture according to any preceeding claim, wherein the hydraulic filler is a mixture of two or more hydraulic fillers.

11. A mixture according to any preceeding claim, wherein the total filler is present in an amount of about 1 - 90 wt.-%, preferably of 30 - 85 wt.-%.

12. A mixture according to any preceeding claim, which contains additional non-salt polymerizable unsaturated monomers or oligomers.

13. A mixture according to claim 1 which contains an additional non-hydraulic inorganic or organic filler or mixtures thereof.

14. Use of a mixture according to any preceeding claim as a pulp capping material, a liner, a dental base material, a cavity lining, a dentin replacement material, a cement for crowns, a cement for bridges, a root canal filling material, an adhesive for brackets or a bone cement.
